(19) Europäisches Patentamt
European Patent Office
Office européen des brevets

(11) Veröffentlichungsnummer: **0 006 205**
**B1**

(12) **EUROPÄISCHE PATENTSCHRIFT**

(45) Veröffentlichungstag der Patentschrift: **22.09.82**

(21) Anmeldenummer: **79101864.1**

(22) Anmeldetag: **11.06.79**

(51) Int. Cl.³: **C 07 C 69/743,**
C 07 C 61/16, A 01 N 53/00

# (54) Verfahren zur Herstellung von Chloro-styryl-cyclopropan-carbonsäure-derivaten.

(30) Priorität: **21.06.78 DE 2827101**

(43) Veröffentlichungstag der Anmeldung:
**09.01.80 Patentblatt 80/1**

(45) Bekanntmachung des Hinweises auf die Patenterteilung:
**22.09.82 Patentblatt 82/38**

(84) Benannte Vertragsstaaten:
**BE CH DE FR GB IT NL**

(56) Entgegenhaltungen:
**DE - A - 2 738 150**

(73) Patentinhaber: **BAYER AG**
**Zentralbereich Patente, Marken und Lizenzen**
**D-5090 Leverkusen 1, Bayerwerk (DE)**

(72) Erfinder: **Diehr, Hans-Joachim, Dr.**
**Höhe 35**
**D-5600 Wuppertal 11 (DE)**
Erfinder: **Fuchs, Rainer Alois, Dr.**
**Röberstrasse 8**
**D-5600 Wuppertal 1 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

EP 0 006 205 B1

Verfahren zur Herstellung von Chloro-styryl-cyclopropan-carbonsäure-derivaten

Die Erfindung betrifft ein neues Verfahren zur Herstellung von teilweise bekannten Chloro-styryl-cyclopropan-carbonsäure-derivaten. Diese können als Zwischen-produkte zur Herstellung von Insektiziden verwendet werden.

Es ist bekannt, daß man Chloro-styryl-cyclopropan-carbonsäureester wie z.B. 3-(2-Chloro-2-phenyl-vinyl)-2,2-dimethyl-cyclopropan-1-carbonsäure-äthylester erhält, wenn man Phenylmethanphosphonsäure-diäthyl-ester mit Butyllithium umsetzt und dann nach einander Tetrachlorkohlenstoff und 3-Formyl-2,2-dimethyl-cyclopropan-1-carbonsäure-äthylester dazu gibt (vergleiche DE - A - 2 738 150).

Dieses Verfahren weist jedoch eine Reihe von Nachteilen auf. Die Umsetzung wird bei —70°C durchgeführt und erfordert somit aufwendige Kühlung. Man benötigt als Base das teure Butyllithium und ein gegenüber Butyllithium inertes Lösungsmittel, welches sorgfältig getrocknet werden muß. Die Aufarbeitung ist langwierig und umfaßt mehrere Extraktionen und Lösungsmitteldestillationen sowie einen chromatographischen Trennungsprozeß. Schließlich erhält man die Produkte als Isomerengemische in unbefriedigenden Ausbeuten.

Daher besteht ein großes Interesse an einer Arbeitsweise, die weitgehend frei von diesen Mängeln ist und die gewünschten Produkte in guten Ausbeuten und hoher Reinheit ergibt.

Es ist weiter bekannt, daß man Chloro-styryl-verbindungen erhält, wenn man $\alpha$-Chloro-benzyl-phosphonsäureester mit Aldehyden in Gegenwart einer Base umsetzt (vergleiche Chimia *28* (1974), 656—657; J. Am.Chem. Soc. *87* (1965), 2777—2778).

Als Base wird hierzu nach dem Stand der Technik Natriumhydrid verwendet, welches ein teueres und schwierig zu handhabendes Reagenz ist. Die Anwendung dieser Synthesemethode ist bisher nur auf wenige Beispiele beschränkt.

Es wurde nun gefunden, daß man Chloro-styryl-cyclopropan-carbonsäure-derivate der Formel I

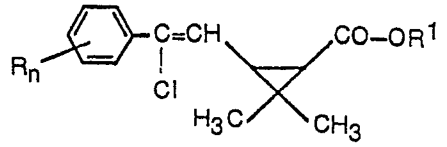

(I)

in welcher
R für Alkyl oder Halogen und
n für 0 bis 5 steht sowie
$R^1$ für Wasserstoff oder Alkyl steht,
durch Umsetzung von $\alpha$-Chloro-benzyl-phosphonsäure-ester der Formel II

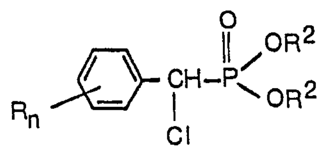

(II)

in welcher
R und n die oben angegebene Bedeutung haben und
$R^2$ für Alkyl oder Phenyl steht oder die beiden Reste $R^2$ zusammen für geradkettiges oder verzweigtes Alkandiyl stehen, mit Formyl-cyclopropan-carbonsäure-derivaten der Formel III

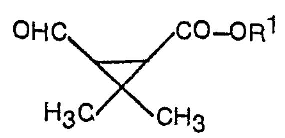

(III)

in welcher
$R^1$ die oben angegebene Bedeutung hat,
gegebenenfalls in Gegenwart von Verdünnungsmitteln bei Temperaturen zwischen —70° und +150°C in guter Ausbeute und hoher Reinheit erhält, indem man in Gegenwart von Alkalialkoholaten oder Alkalihydroxiden arbeitet.

Überraschenderweise können nach diesem neuen Verfahren, welches wesentlich kostengünstiger und einfacher als bekannte Verfahren durchzuführen ist, die erfindungsgemäßen Chloro-styryl-cyclopropan-carbonsäure-derivate in gegenüber dem Stand der Technik verbesserten Ausbeuten erhalten werden.

Als Vorteile des erfindungsgemäßen Verfahrens sind beispielsweise die Möglichkeit der Durchführung bei Raumtemperatur und in wasserhaltigen Reaktionsmedien, die Möglichkeit an Stelle von Butyllithium oder Natriumhydrid relativ billige Basen zu verwenden, die vergleichsweise einfache Aufarbeitung und die guten Ausbeuten zu nennen.

Verwendet man beispielsweise $\alpha$-Chloro-4-fluoro-benzylphosphonsäure-diäthylester und 3-Formyl-2,2-dimethylcyclopropan-1-carbonsäure-methylester als Ausgangsstoffe und Kaliumhydroxid als Hilfsbase, so kann die erfindungsgemäße Reaktion durch folgendes Formelschema skizziert werden:

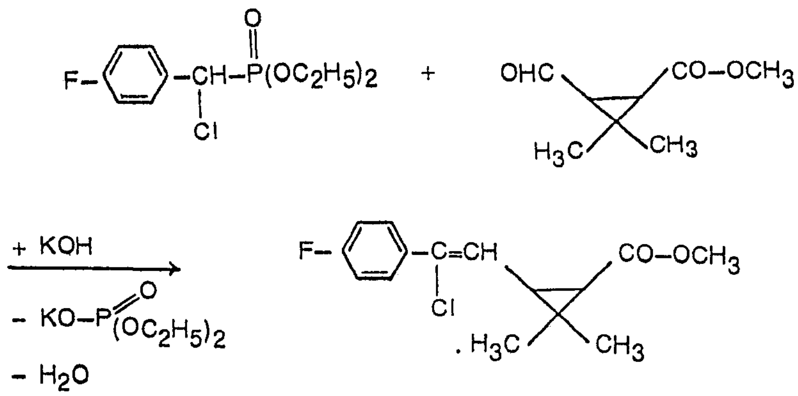


Nach dem erfindungsgemäßen Verfahren werden vorzugsweise Chloro-styryl-cyclopropan-carbonsäure-derivate der Formel (I), in welcher

R für geradkettiges oder verzweigtes Alkyl mit 1 bis 4 Kohlenstoffatomen, insbesondere für Methyl, oder für Fluor, Chlor oder Brom und

n für 0 bis 2 steht, sowie

$R^1$ für Wasserstoff, geradkettiges oder verzweigtes Alkyl mit 1 bis 4, insbesondere 1 oder 2 Kohlenstoffatomen steht, hergestellt.

Die als Ausgangsstoffe zu verwendenden $\alpha$-Chloro-benzyl-phosphonsäureester sind durch Formel (II) definiert.

Vorzugsweise stehen darin

R für geradkettiges oder verzweigtes Alkyl mit 1 bis 4 Kohlenstoffatomen, insbesondere für Methyl, oder für Fluor Chlor oder Brom und

n für 0 bis 2 sowie

$R^2$ für Methyl, Äthyl, Phenyl oder beide Reste $R^2$ stehen zusammen für 2,2-Dimethyl-propan(1,3)-diyl.

Als Beispiele hierfür seien im einzelnen genannt:

$\alpha$-Chloro-benzyl-phosphonsäure-,
$\alpha$-Chloro-4-methyl-benzyl-phosphonsäure-,
$\alpha$-Chloro-4-fluoro-benzyl-phosphonsäure-,
$\alpha$-Chloro-4-chloro-benzyl-phosphonsäure-,
$\alpha$-Chloro-3,4-dichloro-benzyl-phosphonsäure-,
$\alpha$-Chloro-3-bromo-benzyl-phosphonsäure- und
$\alpha$-Chloro-4-bromo-benzyl-phosphonsäure-dimethylester, -diäthylester und -diphenylester.

Die $\alpha$-Chloro-benzyl-phosphonsäureester der Formel (II) sind teilweise bekannt. Sie können durch Umsetzung von $\alpha$-Hydroxy-benzyl-phosphonsäureestern der Formel

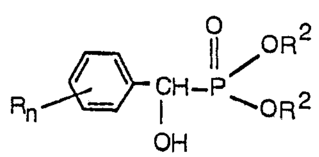 (IV)

in welcher

R, n und $R^2$ die oben angegebene Bedeutung haben, mit einem Chlorierungsmittel, wie z.B. Thionylchlorid oder Phosphoroxychlorid, bei Temperaturen zwischen 0 und 100°C, vorzugsweise bei 20 bis 80°C, hergestellt werden (vergleiche Chimia *28* (1974), 656—657; J.Am.Chem. Soc. *87* (1965), 2777—2778).

Die zur Herstellung der Ausgangsverbindungen der Formel (II) zu verwendenden $\alpha$-Hydroxy-benzyl-phosphonsäure-ester sind durch Formel (IV) definiert.

Vorzugsweise stehen in Formel (IV)

R, n und $R^2$ für diejenigen Reste, welche bereits bei der Definition der Reste in Formel (II) als bevorzugt genannt wurden.

Als Beispiele seien im einzelnen genannt:
α-Hydroxy-benzyl-phosphonsäure-,
α-Hydroxy-4-methyl-benzyl-phosphonsäure-,
α-Hydroxy-4-fluoro-benzyl-phosphonsäure-,
α-Hydroxy-4-chloro-benzyl-phosphonsäure-,
α-Hydroxy-3,4-dichloro-benzyl-phosphonsäure-,
α-Hydroxy-3-bromo-benzyl-phosphonsäure- und
α-Hydroxy-4-bromo-benzyl-phosphonsäure-dimethylester, -diäthylester und -diphenylester.

Die α-Hydroxy-benzyl-phosphonsäureester der Formel (IV) sind teilweise bekannt. Man erhält sie nach bekannten Verfahren, im allgemeinen durch Umsetzung von Aldehyden der Formel

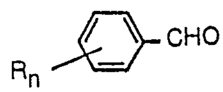

(V)

worin R und n die oben angegebene Bedeutung haben, mit Phosphorigsäureestern der Formel

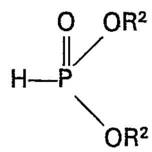

(VI)

worin $R^2$ die oben angegebene Bedeutung hat, gegebenenfalls in Gegenwart eines Katalysators wie z.B. Triäthylamin, bei Temperaturen zwischen 0 und 150°C, vorzugsweise bei 20 bis 100°C (vergleiche Houben-Weyl, Methoden der organischen Chemie, 4. Auflage (1963), Band *12/1,* S. 475—483, Georg Thieme Verlag, Stuttgart).

Als Beispiele für die Aldehyde der Formel (V) seien genannt: Benzaldehyd, 4-Methyl-benzaldehyd, 4-Fluoro-benzaldehyd, 4-Chloro-benzaldehyd, 3,4-Dichlorobenzaldehyd, 3-Bromo-benzaldehyd und 4-Bromo-benzaldehyd.

Die Aldehyde der Formel (V) sind bekannte Verbindungen.

Als Beispiele für die Phosphorigsäureester der Formel (VI) seien genannt: Phosphorigsäure-dimethylester (Dimethylphosphit), Phosphorigsäure-diäthylester (Diäthylphosphit) und Phosphorigsäure-diphenylester (Diphenylphosphit).

Die Phosphorigsäureester der Formel (VI) sind ebenfalls bekannte Verbindungen.

Die weiter als Ausgangsverbindungen zu verwendenden Formyl-cyclopropan-carbonsäurederivate sind durch Formel (III) definiert. Vorzugsweise steht darin
$R^1$ für geradkettiges oder verzweigtes Alkyl mit 1 bis 4, insbesondere 1 oder 2 Kohlenstoffatomen.

Als Beispiele für die Verbindungen der Formel (III) seien im einzelnen genannt: 2,2-Dimethyl-3-formyl-cyclopropan-1-carbonsäure-methylester und -äthylester.

Die Verbindungen der Formel (III) sind bekannt oder können nach bekannten Verfahren, im allgemeinen durch Umsetzung von bekannten Alken(1)yl-cyclopropan-carbonsäureestern mit Ozon hergestellt werden (vergleiche US-A-3 679 667, DE-A-2 621 433 und FR-C-2 281 918).

Das erfindungsgemäße Verfahren zur Herstellung von Verbindungen der Formel (I) wird im allgemeinen unter Verwendung von Verdünnungsmitteln durchgeführt. Als solche kommen praktisch alle inerten organischen Solventien infrage. Hierzu gehören insbesondere aliphatische und aromatische, gegebenenfalls chlorierte Kohlenwasserstoffe, wie Benzin, Benzol, Toluol, Xylol, Methylenchlorid, Chloroform, Tetrachlorkohlenstoff, Chlorbenzol und o-Dichlorbenzol, Äther wie Diäthyl- und Dibutyläther, Tetrahydrofuran und Dioxan. Alkohole wie Methanol, Athanol, n- und iso-Propanol, n-, iso-, sek.- und tert.-Butanol sowie Dimethylsulfoxid.

Bei Arbeiten im Zweiphasenmedium werden neben 50%iger wässriger Natronlauge mit Wasser praktisch nicht mischbare Solventien wie z.B. Benzin, Benzol oder Toluol verwendet.

Als Basen werden Alkalihydroxide wie z.B. Natrium- und Kaliumhydroxid, Alkalialkoholate wie z.B. Kalium- und Natriummethylat, -äthylat, -n- und iso-propylat, -n-, -iso-, -sek.- und tert.-butylat, verwendet.

Vorzugsweise werden als Basen Natrium- und Kaliumhydroxid sowie Natriummethylat und -äthylat verwendet.

Die Reaktionstemperaturen liegen im allgemeinen zwischen −70 und +150°C, vorzugsweise zwischen −10 und +50°C. Die Umsetzung wird im allgemeinen bei Normaldruck durchgeführt.

Zur Durchführung des erfindungsgemäßen Verfahrens werden die Komponenten gewöhnlich in äquimolaren Mengen eingesetzt. Lediglich die Basen werden gewöhnlich in größerem Überschuß eingesetzt: bei Arbeiten im einphasigen System bis zu 30 Mol-%, vorzugsweise bis zu 15 Mol-%; bei Verwendung von 50%iger Natronlauge als zweiter Phase im allgemeinen das 5 bis 15fache der stöchiometrisch erforderlichen Menge.

Alkoholate werden gegebenenfalls in situ aus den Alkoholen und Alkalimetallen hergestellt.

Im allgemeinen wird die Base und gegebenenfalls der Katalysator in einem oder mehreren der genannten Verdünnungsmittel vorgelegt. Dazu werden, gegebenenfalls in einem der angegebenen Solventien gelöst, die Reaktionskomponenten — $\alpha$-Chloro-benzyl-phosphonsäureester und Formyl-cyclopropan-carbonsäure-derivat — in der angegebenen Reihenfolge zugegeben. Zur Vervollständigung der Reaktion wird die Mischung unter Rühren mehrere Stunden im angegebenen Temperaturbereich gehalten.

Zur Aufarbeitung versetzt man die Reaktionsmischung mit Wasser, säuert gegebenenfalls mit Salzsäure an und extrahiert mit Methylenchlorid. Die organische Phase wird getrocknet und das Lösungsmittel wird im Vakuum abdestilliert. Das als Rückstand verbleibende Produkt wird gegebenenfalls durch Vakuumdestillation, oder falls es nicht unzersetzt destillierbar ist, durch sogenanntes "Andestillieren", d.h. durch längeres Erhitzen unter vermindertem Druck auf mäßig erhöhte Temperaturen, gereinigt. Zur Charakterisierung dient der Siedepunkt oder der Brechungsindex.

Verbindungen der Formel (I), worin R, n und $R^1$ die oben angegebene Bedeutung haben, sind bekannt (vergleiche DE - A - 2 738 150).

Solche Verbindungen können als Zwischenprodukte zur Herstellung von insektizid wirksamen Verbindungen der Formel

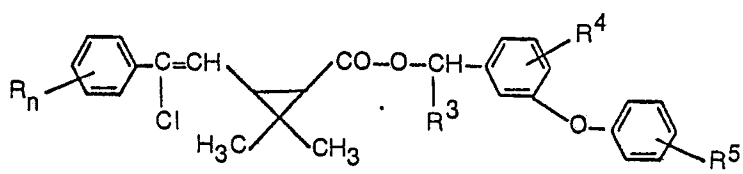

(I a)

in welcher

R und n die oben angegebene Bedeutung haben,

$R^3$ für Wasserstoff, Cyano oder Äthinyl steht sowie

$R^4$ und $R^5$, welche gleich oder verschieden sien können, für Wasserstoff oder Halogen stehen, verwendet werden (vergleiche DE—OS 2 738 150).

Verbindungen der Formel (I a) kann man aus den entsprechenden Zwischenprodukten der Formel (I) auf bekannte Weise herstellen, beispielsweise indem man Chloro-styryl-cyclopropan-carbonsäurealkylester der Formel (I) durch Umsetzung mit wässrig-alkoholischer Natronlauge bei Temperaturen zwischen 20 und 100°C und anschließendes Ansäuern mit Salzsäure zu den entsprechenden Chloro-styryl-cyclopropan-carbonsäuren verseift, daraus durch Umsetzung mit Thionylchlorid in Benzol bei Temperaturen zwischen 20 und 100°C die Carbonsäurechloride herstellt und diese mit Phenoxy-benzylalkoholen der Formel

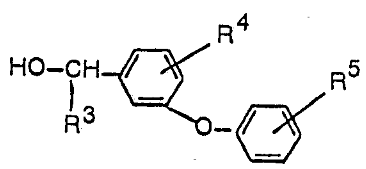

(VII)

in welcher

$R^3$, $R^4$ und $R^5$ die oben angegebene Bedeutung haben, gegebenenfalls in Gegenwart eines Säureakzeptors wie z.B. Pyridin und gegebenenfalls in Gegenwart eines Verdünnungsmittels wie z.B. Benzol, umsetzt.

Die Verwendung der auf diese Weise herzustellenden Produkte als Insektizide ist bekannt (vergleiche DE - A - 2 738 150).

Das erfindungsgemäße Verfahren wird durch folgende Beispiele näher erläutert:

## Herstellungsbeispiele

### Beispiel 1:

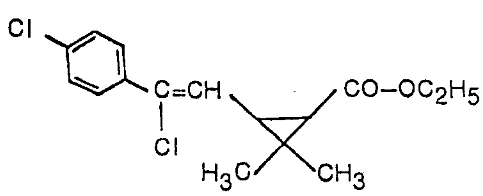

2,53 g (0,11 Mol) Natrium werden portionsweise in 50 ml Äthanol gelöst. Wenn sich alles Natrium aufgelöst hat, werden 150 ml Tetrahydrofuran (wasserfrei) zugesetzt und bei 0°C unter Rühren 29,7 g

(0,1 Mol) 4-Chloro-α-chloro-benzyl-phosphonsäure-diäthylester, gelöst in 30 ml wasserfreiem Tetrahydrofuran, zugetropft. Nachdem noch 2 Stunden bei 0—5°C nachgerührt wurde, werden 17 g (0,1 Mol) cis/trans-2,2-Dimethyl-3-formyl-cyclopropancarbonsäureäthylester, gelöst in 30 ml wasserfreiem Tetrahydrofuran, unter Rühren bei 0°C zugetropft. Anschließend wird noch 12 Stunden bei 20—25°C nachgerührt. Die Reaktionsmischung wird dann mit 500 ml Wasser versetzt und 2 mal mit je 300 ml methylenchlorid extrahiert. Die organische Phase wird abgetrennt, über Magnesiumsulfat getrocknet, das Lösungsmittel im Wasserstrahlvakuum abdestilliert und der Rückstand im Vakuum destilliert. Man erhält 23,2 g (74,1 % der Theorie) 2,2-Dimethyl-3-(2-chloro-2-(4-chloro-phenyl)-vinyl)-cyclopropancarbonsäureäthylester (cis, trans und E, Z-Isomerengemisch) als gelbes Öl mit dem Siedepunkt 150—165°C/1 Torr.

Beispiel 2:

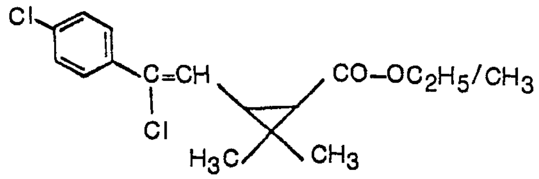

29,7 g (0,1 Mol) 4-Chloro-α-chloro-benzylphosphonsäurediäthylester werden in 150 ml Tetrahydrofuran gelöst und bei 0°C unter Rühren eine Lösung von 6,2 g (0,11 Mol) *Kaliumhydroxid* in 60 ml Methanol zugetropft. Nachdem noch 2 Stunden bei 0 bis 5°C nachgerührt wurde, werden 17 g (0,1 Mol) cis/trans-2,2-Dimethyl-3-formyl-cyclopropancarbonsäure-äthylester, gelöst in 30 ml wasserfreiem Tetrahydrofuran, unter Rühren bei 0°C zugetropft. Anschließend wird noch 12 Stunden bei 20—25°C nachgerührt. Die Reaktionsmischung wird dann mit 500 ml Wasser versetzt und 2 mal mit 300 ml Methylenchlorid extrahiert. Die organische Phase wird abgetrennt, über Magnesiumsulfat getrocknet, das Lösungsmittel im Wasserstrahlvakuum abdestilliert und der Rückstand im Vakuum destilliert. Man erhält 17 g eines Gemisches von 2,2-Dimethyl-3-(2-chloro-2-(4-chloro-phenyl)-vinyl)-cyclopropancarbonsäure-äthyl- und -methylester (cis/trans-, E und Z-Isomere) als gelbes Öl, das im Bereich 145—165°C/1 Torr siedet.

Analog Beispiel 1 oder 2 können die folgenden Verbindungen hergestellt werden:

| Beispiel Nr.: | Produkt | Ausbeute (% der Theorie) | Siedepunkt (°C) | Chem.-Nr. |
|---|---|---|---|---|
| 3 | 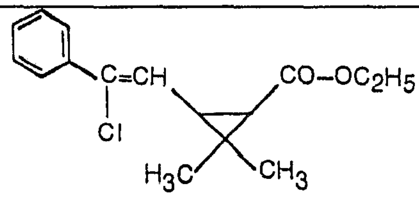 | 68,2 | 140—142/ 2 Torr | |
| 4 | 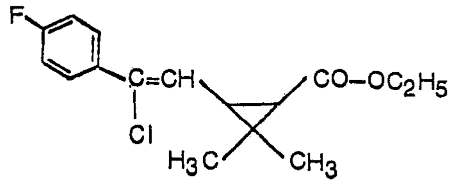 | 58,7 | 135—140/ 2 Torr | |
| 5 | 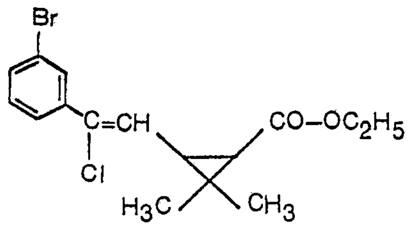 | | | |
| 6 | 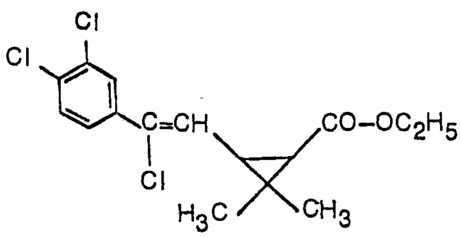 | 62 | $n_D^{25}$:1,5621 | |
| 7 | 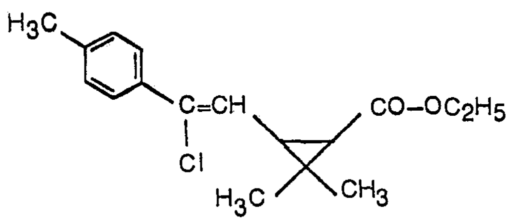 | | | |

Beispiel 8:

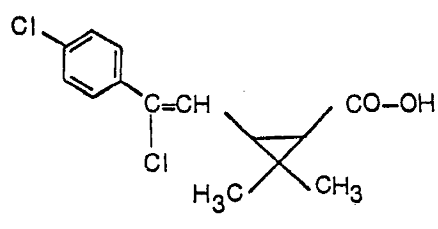

Zu einer Mischung von 100 ml Toluol, 100 ml 50%iger wässriger Natriumhydroxidlösung und 2 g Tetrabutylammoniumbromid werden unter Rühren bei 20—35°C (Eiskühlung) 17 g (0,1 Mol) cis, trans-3-Formyl-2,2-dimethyl-cyclopropancarbonsäureäthylester und 29,7 g (0,1 Mol) $\alpha$-Chloro-4-chloro-benzylphosphonsäurediäthylester, gelöst in 20 ml Toluol, langsam zugetropft. Das Reaktionsgemisch wird dann 12 Stunden bei Raumtemperatur gerührt. Anschließend wird das Reaktionsgemisch in 600 ml Wasser gegossen, die Toluolphase abgetrennt, die wässrige Phase mit konzentrierter Salzsäure angesäuert und anschließend 2 mal mit je 200 ml Methylenchlorid ausgeschüttelt. Die Methylenchloridphase wird abgetrennt, über Magnesiumsulfat getrocknet und

anschließend das Lösungsmittel im Wasserstrahlvakuum abdestilliert. Der verbleibende ölige Rückstand wird destilliert. Man erhält 15,2 g (53,3% der Theorie) 3-(2-Chloro-2-(4-chloro-phenyl)-vinyl)-2,2-dimethyl-cyclopropancarbonsäure als zähes Harz mit einem Siedepunkt von 190—195°C/1 Torr.

Beispiel 9:

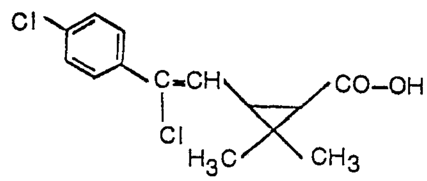

22,2 g (0,071 Mol) 2,2-Dimethyl-3-(2-chloro-2-(4-chloro-phenyl)-vinyl)-cyclopropancarbonsäure-äthylester werden in 100 ml Äthanol gelöst, dann mit einer Lösung von 5,7 g Natriumhydroxid in 100 ml Wasser versetzt und 4 Stunden unter Rühren zum Rückfluß erhitzt. Anschließend wird das Äthanol im Wasserstrahlvakuum abdestilliert, der Rückstand in 300 ml warmen Wasser aufgenommen und 1 mal mit 300 ml Methylenchlorid extrahiert. Die wässrige Phase wird abgetrennt, mit konzentrierter Salzsäure angesäuert und anschließend mit 2 mal 300 ml Methylenchlorid extrahiert. Die organische Phase wird abgetrennt über Magnesiumsulfat, getrocknet und das Lösungsmittel im Wasserstrahlvakuum abdestilliert. Letzte Lösungsmittelreste werden durch kurzes Andestillieren bei 2 Torr/60°C Badtemperatur entfernt. Man erhält dann 15,5 g (76,6 % der Theorie) 2,2-Dimethyl-cyclopropancarbonsäure (cis, und trans, E und Z-Isomerengemisch) zunächst als sehr zähes gelbes Öl, das nach einiger Zeit kristallin erstarrt und dann im Bereich von 94—114°C schmilzt.

Analog Beispiel 9 können die folgenden Verbindungen hergestellt werden:

| Beispiel Nr.: | Produkt | Ausbeute (% der Theorie) |
|---|---|---|
| 10 | 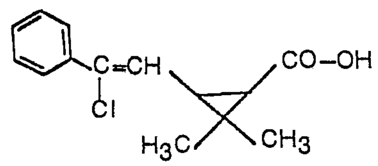 | 68,3 |
| 11 | 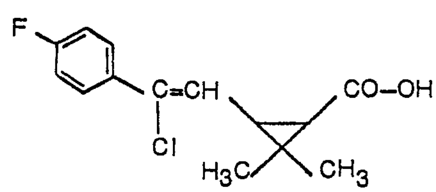 | |
| 12 | 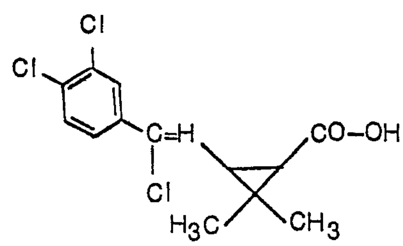 | 72 |

Beispiel 13:

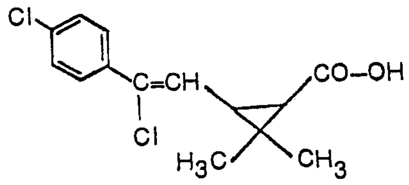

62,6 g (0,2 Mol) 2,2-Dimethyl-3-(2-chloro-2-(4-chloro-phenyl)-vinyl)-cyclopropancarbonsäure-äthylester (Isomerengemisch) werden in 100 ml Äthanol gelöst, dann mit einer Lösung von 10 g Natriumhydroxid in 100 ml Wasser versetzt und 6 Stunden unter Rühren zum Rückfluß erhitzt. Anschließend wird das Äthanol im Wasserstrahlvakuum abdestilliert und der Rückstand mit 150 ml Wasser versetzt. Das Reaktionsgemisch wird dann 12 Stunden auf 5 bis 10°C gekühlt. Anschließend wird der entstandene Niederschlag abgesaugt und getrocknet. Der Feststoff wird dann in 60 ml Methylenchlorid suspendiert und mit 30 ml konzentrierter Salzsäure versetzt. Die organische Phase wird abgetrennt, über Magnesiumsulfat getrocknet und das Lösungsmittel im Wasserstrahlvakuum abgezogen. Man erhält 3,6 g (6,3% der Theorie) cis-2,2-Dimethyl-3-(2-chloro-2-(4-chloro-phenyl)-vinyl)-cyclopropancarbonsäure mit cis-Konfiguration von Phenyl- und Cyclopropanrest an der Doppelbindung als farblose Kristalle vom Festpunkt 140—142°C (aus Äthanol). Die angegebene Konfiguration wird durch das $H^1$NMR und $C^{13}$-NMR-Spektrum bestätigt.

Die als Ausgangsstoffe zu verwendenden $\alpha$-Chloro-benzylphosphonsäureeseter der Formel (II) können wie folgt hergestellt werden:

Beispiel 1a:

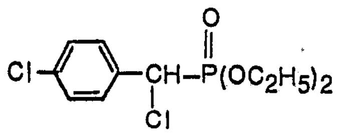

Einem Gemisch aus 20,2 g (0,0725 Mol) 4-Chloro-$\alpha$-hydroxy-benzylphosphonsäure-diäthylester, 65 g Dichloromethan und 5,8 g (0,0725 Mol) Pyridin werden bei 20—40°C unter leichter Wasserkühlung innerhalb von etwa 1 Stunde 9,2 g (0,0768 Mol) Thionylchlorid hinzugesetzt. Das Reaktionsgemisch wird dann 3 Stunden unter Rückfluß erhitzt und 12 Stunden ohne weitere Wärmeeinwirkung nachgerührt. Das Gemisch wird auf etwa 100 g Eiswasser gegossen, die organische Phase abgetrennt und getrocknet. Nach Abdestillieren des Lösungsmittels wird der Rückstand bei 6 Torr und 45°C eingeengt. Man erhält 21 g (97,7% der Theorie) 4-Chloro-$\alpha$-chloro-benzyl-phosphonsäure-diäthylester als gelbes viskoses Öl mit einer Reinheit von 98,6% (Gaschromatogramm) und einem Brechungsindex von $n_D^{24}$: 1,5250.

Analog erhält man die folgenden Verbindungen:

| Beispiel Nr.: | Produkt | Ausbeute (% der Theorie) | Brechungs index: |
|---|---|---|---|
| 3a | 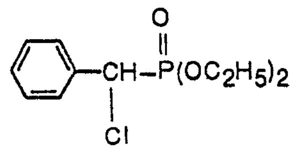 | 93,7 | $n_D^{23}$:1,5117 |
| 4a | 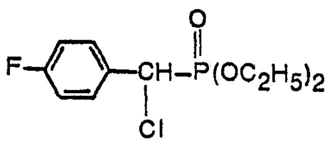 | | |
| 5a | 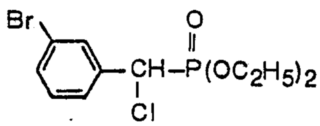 | | |
| 6a | 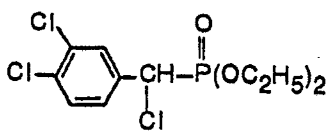 | | |
| 7a | 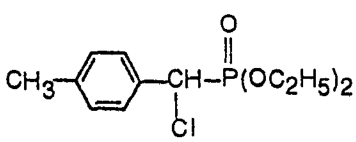 | | |

Die als Vorprodukte benötigten $\alpha$-Hydroxy-benzyl-phosphon-saüreester der Formel (IV) können wie folgt hergestellt werden:

Beispiel 1b:

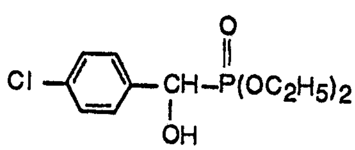

In ein Gemisch aus 20,7 g (0,15 Mol) Diäthylphosphit und 1,09 g (0,0109 Mol) Triäthylamin werden innerhalb einer Stunde unter Wasserkühlung bei 50—70°C 21 g (0,150 Mol) P-Chlorbenzaldehyd eingeleitet. Der Reaktionsansatz wird 1 Stunde bei 70°C nachgerührt. Nach dem Abkühlen wird der Ansatz mit 40 g Toluol aufgenommen und mehrfach mit verdünnter Salzsäure und kaltem Wasser nachgewaschen. Die organische Schicht wird abgetrennt und im Vakuum vom Lösungsmittel befreit. Der feste Rückstand schmilzt bei 70—72°C. Die Ausbeute beträgt 37 g (88,5 % der Theorie) an 4-Chloro-$\alpha$-hydroxy-benzyl-phosphonsäure-diäthylester.

Analog können hergestellt werden:

| Beispiel Nr.: | Produkt | Ausbeute (% der Theorie) | Schmelzpunkt (°C) |
|---|---|---|---|
| 3 b | 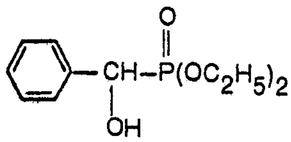 | 88,1 | 75 |
| 4 b | 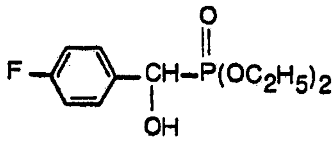 | 94,4 | 26 |
| 5 b | 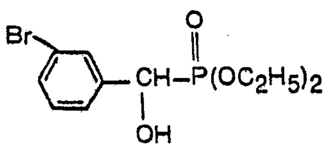 | 86,3 | 42 |
| 6 b | 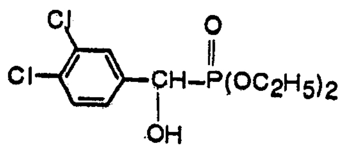 | | |
| 7 b | 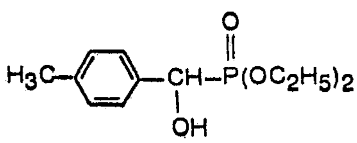 | | |

**Patentansprüche**

1. Verfahren zur Herstellung von Chloro-styryl-cyclopropancarbonsäurederivaten der Formel I

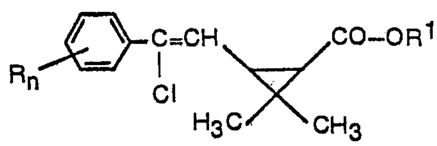

(I)

in welcher
R für Alkyl oder Halogen und
n für 0 bis 5 steht sowie
$R^1$ für Wasserstoff oder Alkyl steht, indem man $\alpha$-Chloro-benzyl-phosphonsäureester der Formel II

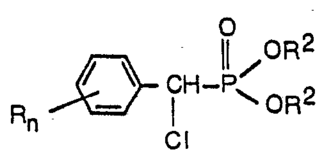

(II)

in welcher
R und n die oben angegebene Bedeutung haben und
$R^2$ für Alkyl oder Phenyl steht oder die beiden Reste $R^2$ zusammen für geradkettiges oder verzweigtes Alkandiyl stehen, mit Formyl-cyclopropan-carbonsäure-derivaten der Formel III

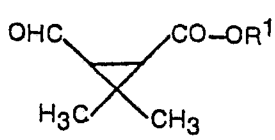

(III)

in welcher
$R^1$ die oben angegebene Bedeutung hat, gegebenenfalls in Gegenwart von Verdünnungsmitteln bei Temperaturen zwischen −70°C und +150°C umsetzt, dadurch gekennzeichnet, daß in Gegenwart von Alkalialkoholaten oder Alkalihydroxiden gearbeitet wird.

2. Verfahren gemäß Anspruch 1, dadurch gekennzeichnet, daß Natrium-oder Kaliumhydroxyd oder Natriummethylat oder -äthylat verwendet wird.

3. Verfahren gemäß Anspruch 1, dadurch gekennzeichnet, daß zwischen −10 und +50°C gearbeitet wird.

4. Verfahren gemäß Anspruch 1, dadurch gekennzeichnet, daß in einem Zweiphasenmedium bestehend aus wässriger Natronlauge und mit Wasser praktisch nicht mischbaren Solvenzien gearbeitet wird.

**Claims**

1. Process for the preparation of chlorostyryl-cyclopropane-carboxylic acid derivatives of the formula I

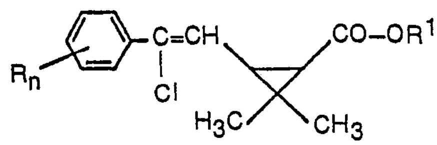

(I)

in which
R represents alkyl or halogen and
n represents 0 to 5 and
$R^1$ represents hydrogen or alkyl, by reacting $\alpha$-chlorobenzyl-phosphonic acid esters of the formula II

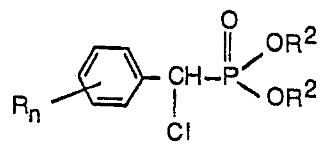

(II)

in which
R and n have the meaning indicated above and
$R^2$ represents alkyl or phenyl or the two radicals $R^2$ together represent straight-chain or branched alkanediyl, with formyl-cyclopropane-carboxylic acid derivatives of the formula III

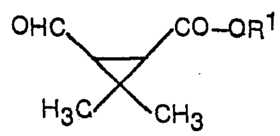

(III)

in which
$R^1$ has the meaning indicated above, if appropriate in the presence of diluents, at temperatures between −70°C and +150°C, characterised in that the reaction is carried out in the presence of alkali metal alcoholates or alkali metal hydroxides.

2. Process according to Claim 1, characterised in that sodium hydroxide or potassium hydroxide, or sodium methylate or sodium ethylate is used.

3. Process according to Claim 1, characterised in that the reaction is effected at between −10 and +50°C.

4. Process according to Claim 1, characterised in that the reaction is effected in a two-phase medium comprising aqueous sodium hydroxide solution and substantially water-immiscible solvents.

**Revendications**

1. Procédé de préparation de dérivés d'acides chloro-styryl-cyclopropane-carboxyliques de formule (I)

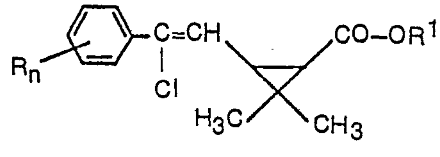

(I)

dans laquelle:
R représente un groupe alkyle ou un halogène,
n est un nombre de 0 à 5, et $R^1$ représente l'hydrogène ou un groupe alkyle, par réaction d'esters $\alpha$-chloro-benzyl-phosphoniques de formule (II)

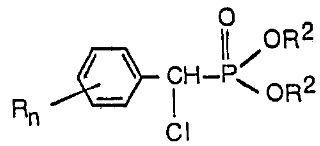

(II)

dans laquelle:
R et n ont les significations indiquées ci-dessus, et
$R^2$ représente un groupe alkyle ou phényle ou bien les deux symboles $R^2$ représentent ensemble un groupe alcane-diyle à chaîne droite ou ramifiée, avec des dérivés de l'acide formyl-cyclopropane-carboxylique de formule (III)

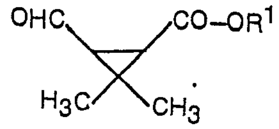

(III)

dans laquelle $R^1$ a la signification indiquée ci-dessus, éventuellement en présence de diluants, à des températures allant de −70 à +150°C, caractérisé en ce que l'on opère en présence d'alcoolates alcalins ou d'hydroxydes alcalins.

2. Procédé selon la revendication 1, caractérisé en ce que l'on utilise l'hydroxyde de sodium ou de potassium ou le méthylate ou l'éthylate de sodium.

3. Procédé selon la revendication 1, caractérisé en ce que l'on opère entre −10 et +50°C.

4. Procédé selon la revendication 1, caractérisé en ce que l'on opère dans un milieu en deux phases consistant en lessive de soude et solvants pratiquement non miscibles à l'eau.